# EUROPEAN PATENT SPECIFICATION

(11) **EP 2 117 452 B1**
(45) Date of publication and mention of the grant of the patent: **20.07.2016**
(21) Application number: 08725496.7
(22) Date of filing: 12.02.2008
(51) Int. Cl.: A61B 17/74, A61B 17/80, A61B 17/82, A61B 17/84

(54) **TROCHANTERIC GRIP**
TROCHANTÄRER GRIFF
POIGNÉE TROCHANTÉRIENNE

(30) Priority: 13.02.2007 US 706513
(43) Date of publication of application: 18.11.2009
(73) Proprietor: Kinamed, Inc., Camarillo, CA 93012 (US)
(72) Inventor: SARIN, Vineet, K., Simi Valley, CA 93065 (US); NICHOLSON, James, J., Setauket, NY 11733 (US); KENDALL, Richard, L., Oak View, CA 93022 (US); PRATT, William, R., Newbury Park, CA 93021 (US)
(74) Representative: Gevers Patents
(86) International application number: PCT/US2008/001871
(87) International publication number: WO 2008/100525

(56) References cited:
- US-A- 4 269 180
- US-A- 5 607 430
- US-A- 5 665 089
- US-A- 5 665 089
- US-A1- 2006 058 795
- US-A1- 2006 058 795
- US-A1- 2006 235 401

## Description

### BACKGROUND OF THE INVENTION

### Field of the Invention

This invention relates generally to surgical implants used in orthopedic surgery, and more specifically to an apparatus grip useful in the attachment of a greater trochanter.

### Description of the Related Art

Hip replacement surgery has become commonplace. During a revision of a total hip replacement, the greater trochanter is commonly cut away from the femur and retracted, together with the abductor musculature to which the trochanter is attached. This greatly facilitates the surgical approach to the hip joint. A femoral stem is then replaced by new prosthetic implant. This technique is also employed in connection with trochanteric osteotomies and intra-operative fractures of the trochanter.

After the implant is located, the greater trochanter is relocated and must be reattached. To properly heal, the greater trochanter must be secured in its proper position on the proximal femur and the position maintained for a time sufficient for the bone to heal. Maintaining the proper position is difficult because of the very substantial and dynamic forces applied to the trochanter and femur, both through the femur and from the attached abductor musculature, which tends to move the trochanter in relation to the femur.

Various bone plates or grips have been introduced to secure the trochanter during healing. One such device is described in U.S. Patent 6,066,141, for example. Other examples are described in U.S. patents 6338734; 5993452; 5797916; 5665088; 5334291; 4889110; and 4269180. A more recent example of a trochanteric cerclage plate is published in U.S. published application 2006058795. Typically the prior devices have a metallic body with one or more grooves or bores through which cables may be threaded. The cable is passed around the femur and fixed in tensioned loops, clamping the trochanter in place on the proximal femur. Some configurations require drilling holes through the femur, through which the cable is passed.
US 2006/0235401 A1 discloses a method and apparatus for repairing a femur. A connector is provided having a claw-like member to engage with the greater trochanter. Along the body of the connector are a plurality of cable apertures and cable screws to receive and engage with cables that loop around the femur. Along the inferior end of the connector are bone screw slots and bone screws engaging the connector with the femur. The bone screws provide added support to the re-attached greater trochanter and provide support for periprosthetic fractures. The connector may be used to re-attach the greater trochanter by impacting a connector onto the greater trochanter, positioning the greater trochanter onto the femur, passing cables around the femur and through the connector, tensioning the cables to provide engagement between the greater trochanter and the femur, and attaching the connector to the femur using at least one bone screw.

These and other prior designs have cable retention features such as grooves or bores arranged in ways that require the cable to make abrupt bends or curves, in some cases crossing abrupt ledges or sharp corners.

### SUMMARY OF THE INVENTION

The present invention provides a bone plate as claimed in claim 1.

These and other features and advantages of the invention will be apparent to those skilled in the art from the following detailed description of preferred embodiments, taken together with the accompanying drawings, in which:

### BRIEF DESCRIPTION OF THE DRAWINGS

FIG. 1 is a perspective view of a bone grip in accordance with the invention;
FIG. 2 is a top view of the bone grip of FIG. 1;
FIG. 3 is a side view of the bone grip of FIGs 1 and 2;
FIG. 4 is a cross section taken along section line 4 in FIG 3;
FIG. 5 is a cross section taken along Section line 5 in FIG. 3;
FIG. 6 is a cross section taken along Section line 6 in FIG. 3;
FIG. 7 is a cross section taken along section line 7 in FIG. 3; and
FIG. 8 is a perspective view of the bone grip positioned in relation to a human femur, and showing cable attachments according to a method of using the device in reattachment of a greater trochanter.

### DETAILED DESCRIPTION OF THE INVENTION

The terms "proximal" and "distal" as used herein are defined for convenience in relation to the intended anatomical orientation of the device when surgically fixed to a greater trochanter. However, in some applications the device might be reoriented without departing from the invention. Accordingly, the terms proximal and distal should be understood only as convenient labels for the purpose of description, and not as limiting the possibilities for reorientation.

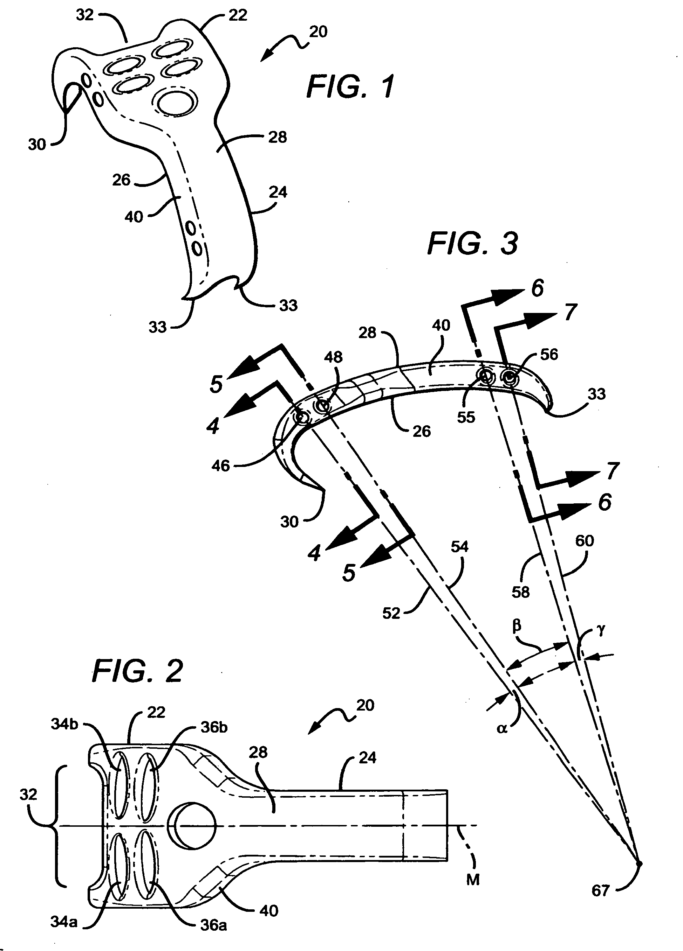

As shown in FIGs 1-3, a bone grip in accordance with the invention has a solid, substantially rigid body 20 with a relatively wider proximal portion 22 and a relatively more narrow distal neck portion 24. An inner face 26 of the body 20 is slightly concave in at least the proximal-distal direction, conforming to a generalized surface of a greater trochanter with its soft tissue attachments (or other bone, as required by each particular embodiment). An outer face 28 of the body 20 approximately follows the contour of the inner face on the opposite side of the thickness of the body. The overall shape of the body is thus generally shell-like, with its inner face cupped slightly inward to embrace a convex bony surface.

The body 20 preferably has a length dimension longer than the width dimension, defining a lengthwise direction. The body 20 also preferably has generally bilateral symmetry so that an imaginary midplane or medial plane of symmetry is defined. The medial plane M of the body extends in the direction of the longer dimension (lengthwise) of the body and is disposed approximately midway across the width dimension. Because the body 20 is intended to be fixed to a femur with the long dimension extending from proximal to distal (in anatomical terms), the M plane will normally be fixed to extend in the proximal-distal direction in anatomical relation to the femur. The medial plane M intersects outer face 28 to define an imaginary midline.

At the proximal end of Body 20 at least two proximal, hooked tines 30 are separated by a gap 32. The tines are generally sharp, to securely engage into or over the proximal bony surface of a greater trochanter. Similarly, at the distal end of the body 20, the distal end has at least two smaller hooked, distal tines 33 sharpened to engage a femur (or other similar bone). The tines are preferably disposed symmetrically. Thus, the medial plane M lies midway between the proximal tines 30, midway between the distal tines 33, and generally normal to the inner and outer faces 26 and 28.

Referring to FIG. 2, the body 20 has at least two, and preferably a larger number of oblique bores 34a, 34b, 36a and 36b (generally identified as oblique bores). The bores are aligned in convergent, opposed pairs. In the illustrated example, bores 34a and 34b converge at an apex near the medial plane M. Each bore-pair comprises a cable channel, as discussed in detail below. Preferably, at least a second set of convergent, opposed bores 36a and 36b are also provided; the second set together comprise a second cable channel.

The oblique bores run generally transverse to the axis of the body 20, entering from a side 40 and exiting the top surface 28. It should be borne in mind that in a preferred embodiment with sides 40 and top surface nearly perpendicular, the bores will be non-perpendicular with either the top surface 28 or the sides 40. As shown in FIG. 2, the intersection of bores 34a, 34b, 36a and 36b with the top surface 28 is acutely oblique, creating an aperture which appears elliptical (although it is not mathematically an exact ellipse). The intersection with the sides 40 is also non-perpendicular, yielding a non-circular side aperture 46 but with less eccentricity than the upper apertures where bores 34a,b and 36a,b exit the top surface 28.

Although only four lateral bores are visible from the side view of FIG. 3, it will be seen from the other views and from the approximately bilateral symmetry of the body that right and left bores are provided in sets, generally transverse to the medial plane M body 20 and lining up in pairs. Each pair of corresponding right and left bores together comprises a cable guide, having diameter complementary to a matching strand of cerclage cable. These cable guides are disposed to cooperate with surgical cerclage cables to allow fixation of the bone grip in the manner discussed below.

The cross-section 4 shows that the left and right lateral bore pair 34a and 34b are generally transverse to the medial plane of the body 20, but are neither parallel nor skew to one another. Rather, the bores are angled upwards, converging toward an intersection at or above an apex approximately at the medial plane of the body 22. Preferably, the central axes of bores 34a and 34b intersect near the top surface 28. The bores thus form complementary ramps sloping downward and outward from a central apex at 50. In accordance with the invention, the axes of these ramps intersect at an obtuse angle θ. A curved transition between ramps is preferably provided at the apex 50.

Inasmuch as the axes of these bores 34a and 34b intersect at an obtuse angle, they define a plane (the "cable guide plane"). Two intersecting lines define a plane, as is well known (Euclid). Therefore, the cable guide comprising bores 34a and 34b lies generally on and defines a first cable guide plane 52, as shown in FIG. 3. Each cable guide plane is transverse to the medial plane M; but multiple ones of the cable guide planes are not necessarily parallel to one another, as discussed further below and as seen in FIG. 3.

It is preferred that the ridge separating bore 34a and 34b be slightly rounded to a saddle-like shape, to soften the transition for a cable running through 34a and 34b, passing across the apex ridge. This can be manufactured, for example, by threading a strong, abrasive-impregnated cable through the cable guide comprising bore pair 34a and 34b. The cable is then tensioned and pulled alternately back and forth through the channel while maintaining tension, to abrade the body and define a smooth saddle or cable groove.

The ramps defined by 34a and 34b are preferably disposed at obtuse angles in relation to a desired direction of cable tension. The angle of cable tension is defined by the anatomy of the femur, and in particular the relationship between the greater and lesser trochanter. In consideration of this anatomical relationship, the arrangement of the invention tends to distribute cable stress by avoiding any acute or right-angle corners. Note that the angle ϕ between the left face 40 of the device and the bore 34a is preferably more than 90 degrees.

The transition θ between ramps again presents an obtuse angle; another obtuse angle is formed at the aperture of the right hand bore 34b in right face. The series of obtuse angles tends to distribute the contact stress across ramps 34a and 34b so that stress is distributed in a cable that is passed through the guides.

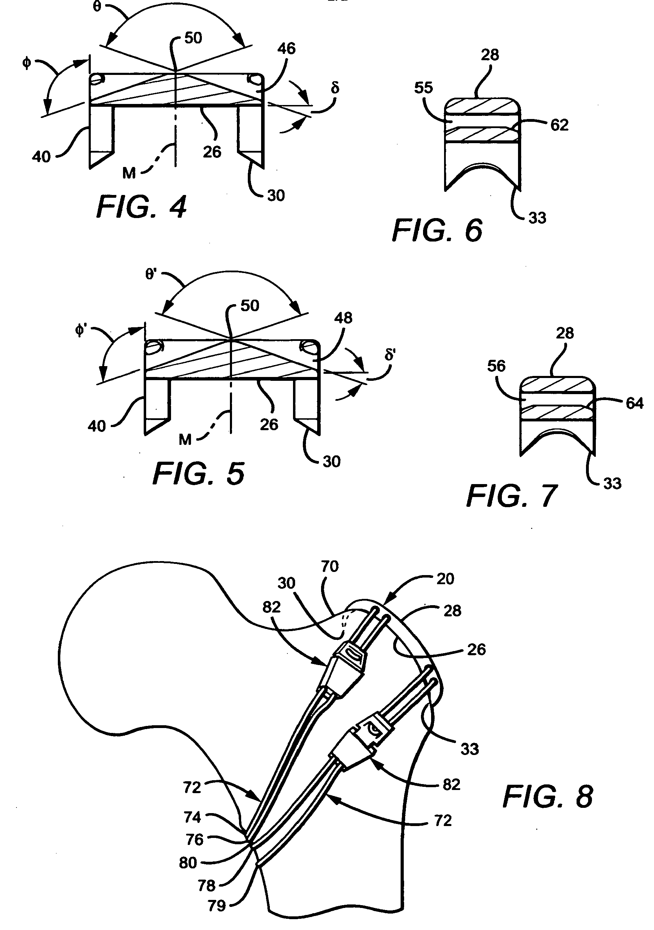

Similarly, FIG. 5 shows a second cable guide analogous to that of FIG. 4. However, cut plane 5 (54 in FIG. 3) is not parallel with that of FIG. 4 (52 in FIG. 3). The Cut planes 54 and 52 are defined by the directions of their corresponding cable guides. As discussed above, each pair of bores (34a,b; 36a,b) are non-parallel, convergent and intersecting near an apex. Thus, each pair defines a plane. In accordance with the invention, planes 52 and 54 intersect as shown at a dihedral angle a which, in a preferred embodiment, is approximately three degrees. The actual angle in a give embodiment is determined by the desired anatomical fixation point at which the cables are intended to converge. Thus, the angle is predetermined such that the cable guide planes converge near an anatomical fixation point (for example, the lesser trochanter) when the plate is fixed on the greater trochanter.

It should be noted that the angles θ',φ' in FIG. 5 may vary slightly from corresponding angles θ, φ in FIG.4, depending on anatomical geometry for the particular application, but preferably both will be obtuse, as discussed above.

In the more distal neck 24 of the bone grip, at least one, and preferably at least two more directed cable guides 55 and 56 are provided. In one embodiment, bore pairs similar to 34a,b and 36a,b are provided at 55 and 56 in the neck 24, each defining a different plane 58 and 60. (differing from one another and from plane 4 and plane 5). Alternatively, through bores 55 and 56 can be used, as illustrated in the figures. The alternative arrangement is more easily fabricated in a body having a neck narrower than the proximal body, as shown. This alternative is illustrated because the arrangement of 34a,b and 36a,b has already been shown and described.

Cross sections 6 and 8 show bores 55 and 56, respectively. Although the bores are generally tranverse and pass through the body, each side is preferably counterbored at an oblique angle. The directions of the oblique counterbores provide in each bore a short ramp (shown at 62 and 64) which together with the bores defines cable guide planes 58 and 60. Alternatively, the counterbore may simply be chamfered or rounded to avoid concentration of stress in the cable. The apertures of all bores should be smoothed as by abrasion to a finish, for example 32 Ra (microinches) (0.8µm) rms roughness, to prevent abrasion of an elastic, polymer cable.

Referring back to FIG. 3, one can see that the planes 58 and (optional) 60 are not parallel to one another or to either 52 or 54. Preferably the dihedral angle β between the (innermost of the) proximal cable guide planes and the distal cable guide planes is approximately 22 degrees. More generally the angle could be in the range 10 to 50 degrees. This angle is, in any particular embodiment, determined by trochanter geometry more specifically, the angle is selected such that the cable guide planes converge at or near a predetermined fixation point (for example, the lesser trochanter). In this context, "near" is used to mean within 2 centimeters. The angle γ between planes of the two distal neck cable guides is approximately three degrees, but may vary in response to the relative locations of the greater trochanter and fixation point in a given anatomical context. Preferably, all of the planes defined by the multiple cable guides intersect at approximately the same line seen (pointing into the page) as 67, which corresponds with an estimated attachment point (suitably at or below the lesser trochanter). This arrangement directs all the tension vectors in a cerclage cable in the most anatomically and mechanically desirable directions, and avoids unnecessary kinks and opportunity for cable abrasion.

FIG. 8 shows an example of a method of use for the trochanteric grip of the invention. The device 20 is disposed in contact with a greater trochanter at 70, with the concave inner face 26 disposed toward the bone, and the convex outer face 28 disposed outward and upward. The tines 33 and 30 are anatomically arranged so that the device naturally seats with the medial plane M running generally in the proximal-distal direction in relation to the patient's anatomy. Two lengths of elastic, polymer cable 72 are doubled to provide four strands 74, 76, 78 and 79. Each strand is passed through a cable guide: 74 passes through the bore pair 34a and 34b (comprising guide 34); 76 passes through guide 36, and so on. The cable is passed around the femur, preferably passing through or below the lesser trochanter; and the free ends are secured under tension.

One method of securing the cable is shown: The looped end and the free ends can be secured under tension by a pair of locking, wedged cable clamps 82. An example of a suitable clamp is described in U.S. Patent application 11/147685 filed on 6/08/2005 (allowed, pending issue). Other means could be employed for securing the cable under tension. Two such cable segments (four strands) are shown and are preferred.

As shown in FIG. 8, the various strands of cable are not directed in parallel or in the same plane; rather, the loops tend to converge at 80 to engage the lesser trochanter. This arrangement is preferred by the surgeon for the security that it offers, and because it tends to direct the force vectors in the direction most preferred to maintain dynamic compression on the trochanter during post-surgical recovery period. This tends to promote proper healing and prevent post-operative displacement of the trochanter, which experiences large dynamic and static stresses from the muscle attachments (not shown, to maintain clarity of illustration).

The cable guides of the present invention are preferably directed in convergent planes that follow the tension directed in a tensed, elastic cable looped through the trochanteric grip (at a first extreme of the loop) and secured around the femur at the lesser trochanter (at the opposite extreme of the loop).

Numerous variations of the apparatus and method are possible. Optionally, a hole, notch, or other feature may be provided on the body 20 of the invention for engaging a complementary instrument for manually manipulating the body during surgery. For example, a threaded hole may optionally be provided in body 20 , for fitting to a complementary threaded shaft on an instrument. A shaft might alternatively be press fitted, or notches, projections, or recesses of various forms might be provided, depending on the specific design of the complementary handling instrument. In some instances of the method, holes may be drilled through the lesser trochanter and the cables threaded through; in other cases, it may be sufficiently secure to rely on the protrusion of the lesser trochanter to retain the cable loop (as shown in FIG. 8).

While several illustrative embodiments of the invention have been shown and described, numerous variations, additions, and alternate embodiments will occur to those skilled in the art. Such variations and alternate embodiments are contemplated, and can be made without departing from the scope of the invention as defined in the appended claims.

## Claims

1. A bone plate for use in fixing a resected bony piece to a larger bone, suitable for fixing a greater trochanter to a femur, comprising:
a body (20) having a proximal end and a distal end disposed at opposite ends of a lengthwise dimension, said body (20) also having an outer face (28) and an inner face (26);
said body having first and second cable guides (55, 56), said first and second cable guides comprising first and second pairs of sloping ramps, respectively, each pair of sloping ramps converging at an obtuse angle to intersect, said first and second pairs of sloping ramps defining first and second cable-guide planes (52, 54);
wherein said first and second cable guide planes (52, 54) are disposed generally transverse to the lengthwise dimension of said body, **characterised in that** said first and second cable guide planes are not parallel to each other, and intersect at a dihedral angle (α).

2. The bone plate of claim 1, wherein each of said pairs of sloping ramps comprises a pair of bores (34a, 34b; 36a, 36b) directed generally transverse to said lengthwise dimension of said body (20) and oblique to said outer face of said body, each of said pairs of bores converging to intersect near a midplane defined by said body (20).

3. The bone plate of claim 1, wherein said cable-guide planes (52, 54) intersect at a line predetermined to pass near an anatomical fixation point.

4. The bone plate of claim 3, wherein said cable-guide planes are arranged to direct cable tension in convergent planes converging in a line of intersection, said line of intersection disposed generally transverse to said midplane of said body.

5. The bone plate of claim 2, further comprising at least one further bore capable of receiving a cable (72).

6. The bone plate of claim 5, wherein said further bore has at least one oblique counterbore, said oblique counterbore and said bore defining a third cable guide plane, transverse to said lengthwise dimension and non-parallel with either of said first and second cable guide planes.

7. The bone plate of Claim 6, wherein said third cable guide plane is disposed at a dihedral angle in the range from 10 to 50 degrees.

8. The bone plate of claim 5, wherein said further bore has a chamfered aperture.

## Patentansprüche

1. Knochenplatte zur Verwendung bei der Befestigung eines herausgeschnittenen knochigen Stücks auf einem breiteren Knochen, die sich zur Befestigung eines größeren Trochanters an einem Oberschenkel eignet, folgendes umfassend:
einen Korpus (20), der ein proximales Ende und ein distales Ende aufweist, die an gegenüberliegenden Enden einer Längsabmessung angeordnet sind, wobei der besagte Korpus (20) auch eine Außenfläche (28) und eine Innenfläche (26) aufweist;
wobei der besagte Korpus erste und zweite Kabelführungen (55, 56) aufweist, wobei die besagten ersten und zweiten Kabelführungen jeweils erste und zweite Paare an schrägen Rampen umfassen, und jedes Paar an schrägen Rampen in einem stumpfen Winkel zu einem Schnittpunkt zusammenlaufen, wobei die besagten ersten und zweiten Paare an schrägen Rampen erste und zweite Kabelführungsebenen (52, 54) definieren;
wobei die besagten ersten und zweiten Kabelführungsebenen (52, 54) im Allgemeinen quer zur Längsabmessung des besagten Korpus angeordnet sind, **dadurch gekennzeichnet, dass** die besagten ersten und zweiten Kabelführungsebenen nicht parallel zueinander liegen und sich in einem Öffnungswinkel (α) schneiden.

2. Knochenplatte nach Anspruch 1, wobei jedes der besagten Paare an schrägen Rampen ein Paar an Bohrungen (34a, 34b; 36a, 36b) umfasst, die im Allgemeinen quer zur Längsabmessung des besagten Korpus (20) und schräg zur besagten Außenfläche des besagten Korpus ausgerichtet ist, wobei jedes der besagten Paare an Bohrungen zusammenläuft, um sich nahe einer Mittelebene zu schneiden, die vom besagten Korpus (20) definiert wird.

3. Knochenplatte nach Anspruch 1, wobei sich die besagten Kabelführungsebenen (52, 54) in einer Linie schneiden, die vorbestimmt ist, um nahe eines anatomischen Befestigungspunkts vorbeizuführen.

4. Knochenplatte nach Anspruch 3, wobei die besagten Kabelführungsebenen angeordnet sind, um die Kabelspannung in zusammenlaufende Ebenen zu leiten, wobei die besagte Schnittlinie im Allgemeinen quer zur besagten Mittelebene des besagten Korpus angeordnet ist.

5. Knochenplatte nach Anspruch 2, darüber hinaus zumindest eine weitere Bohrung umfassend, die imstande ist, ein Kabel (72) aufzunehmen.

6. Knochenplatte nach Anspruch 5, wobei die besagte weitere Bohrung zumindest eine schräge Senkung aufweist, wobei die besagte schräge Senkung und die besagte Bohrung eine dritte Kabelführungsebene definieren, die quer zur besagten Längsabmessung und nicht parallel zu den beiden besagten ersten und zweiten Kabelführungsebenen verläuft.

7. Knochenplatte nach Anspruch 6, wobei die besagte dritte Kabelführungsebene in einem Öffnungswinkel in einem Bereich von 10 bis 50 Grad angeordnet ist.

8. Knochenplatte nach Anspruch 5, wobei die besagte weitere Bohrung eine gefaste Öffnung aufweist.

## Revendications

1. Lame osseuse pour utilisation dans la fixation d'une pièce osseuse réséquée à un os plus grand, convenant à la fixation d'un grand trochanter à un fémur, comprenant :
un corps (20) ayant une extrémité proximale et une extrémité distale disposées à des extrémités opposées d'une dimension longitudinale, ledit corps (20) ayant également une face externe (28) et une face interne (26) ;
ledit corps ayant des premier et second guides de câble (55, 56), lesdits premier et second guides de câble comprenant des première et une paire de rampes inclinées, respectivement, chaque paire de rampes inclinées convergeant selon un angle obtus pour avoir une intersection, lesdites première et seconde paires de rampes inclinées définissant des premier et deuxième plans de guides de câble (52, 54) ;
dans laquelle lesdits premier et deuxième plans de guides de câble (52, 54) sont disposés de manière généralement transversale à la dimension longitudinale dudit corps, **caractérisée en ce que** lesdits premier et deuxième plans de guides de câble ne sont pas parallèles l'un à l'autre et se coupent selon un angle dièdre (α).

2. Lame osseuse selon la revendication 1, dans laquelle chacune desdites paires de rampes inclinées comprend une paire de trous (34a, 34b ; 36a, 36b) dirigés de manière généralement transversale à ladite dimension longitudinale dudit corps (20) et en oblique vis-à-vis de la face externe dudit corps, chacune desdites paires de trous convergeant pour se couper vers un plan moyen défini par ledit corps (20).

3. Lame osseuse selon la revendication 1, dans laquelle lesdits plans de guides de câble (52, 54) se coupent sur une ligne prédéterminée pour passer vers un point de fixation anatomique.

4. Lame osseuse selon la revendication 3, dans laquelle lesdits plans de guides de câble sont aménagés pour diriger une tension de câble dans des plans convergents qui convergent sur une ligne d'intersection, ladite ligne d'intersection étant disposée de manière généralement transversale audit plan moyen dudit corps.

5. Lame osseuse selon la revendication 2, comprenant en outre au moins un autre trou capable de recevoir un câble (72).

6. Lame osseuse selon la revendication 5, dans laquelle ledit autre trou a au moins un contre-trou oblique, ledit contre-trou oblique et ledit trou définissant un troisième plan de guides de câble transversal à ladite dimension longitudinale et non parallèle à l'un ou l'autre desdits premier et deuxième plans de guides de câble.

7. Lame osseuse selon la revendication 6, dans laquelle ledit troisième plan de guides de câble est disposé selon un angle dièdre dans la plage de 10 à 50 degrés.

8. Lame osseuse selon la revendication 5, dans laquelle ledit autre trou a une ouverture biseautée.
